# EUROPEAN PATENT SPECIFICATION

(11) **EP 1 432 464 B1**
(45) Date of publication and mention of the grant of the patent: **14.01.2009**
(21) Application number: 02799393.0
(22) Date of filing: 23.09.2002
(51) Int. Cl.: A61M 5/168

(54) **MODULAR DRUG DELIVERY SYSTEM**
MODULARE VORRICHTUNG ZUR VERABREICHUNG VON MEDIKAMENTEN
SYSTEME D'ADMINISTRATION DE PRODUIT MODULAIRE

(30) Priority: 26.09.2001 DK 200101403
(43) Date of publication of application: 30.06.2004
(73) Proprietor: NOVO NORDISK A/S, 2880 Bagsvaerd (DK)
(72) Inventor: POULSEN, Jens, Ulrik, DK-2830 Virum (DK); KRAG-JENSEN, Christian, DK-2100 Copenhagen (DK)
(86) International application number: PCT/DK2002/000616
(87) International publication number: WO 2003/026726

(56) References cited:
- EP-A- 0 118 008
- WO-A-98/00186
- WO-A-98/34657
- US-A- 4 652 261
- US-A- 5 474 527

## Description

The invention relates to a modular drug delivery system, comprising a reservoir unit and a control unit in combination with at least one further control unit, whereby each combination of a reservoir unit and a control unit provides different capabilities. The system may be adapted for subcutaneous or intra-dermal infusion of a liquid formulation of an active ingredient such as insulin.

A modular system comprising the features defined in the preamble of claim 1 is disclosed in WO-A-98/34657.

### BACKGROUND OF THE INVENTION

In the disclosure of the present invention reference is mostly made to the treatment of diabetes by infusion of insulin, however, this is only a preferred use of the present invention.

By treatment of certain medical conditions, such as insulin treatment of type 1 or 2 diabetes, either a.conventional syringe can be used, or an injection device into which a cartridge containing the drug to be used is contained. The best know and mostly used injection device for this purpose is "pen"-formed devices typically used for treatment of diabetes types 1 or 2, where the pen can be considered a manually actuated pump serving as the infusion means for the cartridge.

A different class of devices is in the form of automatic infusion pump devices, also known as infusers, which are devices carried by and connected to the user through a catheter or hypodermic needle. In its simplest form the infuser will provide continuous delivery of the drug at a desired rate, however, more recent infusers can be programmed to infuse the drug in accordance with any desired infusion profile, just as the user manually can modify the infusion profile by, for example, adding bolus doses in relation to the meats.

The pump offers the possibility of good control of blood glucose concentration as it may simulate the course of the insulin production by a non-diabetic. However, the use of insulin pumps has been rather limited as the pumps have been expensive to manufacture as well as complex to operate. In an attempt to provide improved user friendliness, an early infuser used a cartridge similar to the ones used in the pen devices which meant that loading of the cartridge into the pump mechanism was rather cumbersome just as the need to keep track of the batteries not running flat added to the complexity experienced by the user. However, later infusers have mostly been designed with reservoirs which have to be filled by the user just prior to use.

Further, as more and more programming features were added, this complexity alone made the infusers unsuitable for certain group of users.

Consequently, infusers were developed which tried to alleviate some of these drawbacks. A first type of devices addressing these problems had in common the use of some kind of disposable "cassette" comprising some further means in addition to the mere reservoir, for example the so-called "infusion set" comprising the means for introducing the drug, e.g. an integrated or pre-connected catheter and/or infusion needle.

An early example can be found in WO 85/00523 teaching to integrate the power source for the pump into a disposable unit, however, the disposable unit further comprises an infusion line for insertion into a pump head to provide a peristaltic pump, this complicating the mounting of the disposable unit.

The problem of mounting a cassette comprising an infusion line for a peristaltic pump is addressed in FR-A-2 753 103 disclosing an infusion system comprising a durable pump portion and a click-on cassette portion.

US 4 886 499 discloses a portable device which in a preferred embodiment comprises a disposable unit which can be mounted on a reusable unit in a click-on fashion, the disposable unit comprising the drug containing reservoir as well as the pump, however, the battery is arranged in the reusable unit.

By integrating even more disposable parts in a single disposable cassette unit the following generations of infusers were made increasingly simple to handle, yet providing improved functionality of the infuser *per se*. For example, US 5 984 894 discloses an infusion system comprising a durable unit forming a housing and a disposable unit containing the liquid reservoir and an energy reservoir for energizing the pumping function together with all liquid-contacting elements of the device. The durable unit is in the form of a housing comprising an inner space into which the disposable unit is adapted to be inserted after which a lid is closed.

On the basis of the above-described two-unit system, US 5 984 894 discloses that the mechanic and/or electronic interface between the disposable and durable unit can be selected in accordance with the actual circumstances. Accordingly, the durable unit may comprise one or more of the elements: A controlling unit, a display, means for setting the controlling unit, a drive unit delivering mechanical energy for driving the pump mechanism and a long life electric cell energizing the controlling unit. The pumping mechanism may be a complete low cost pump, e.g. a piezoelectric membrane pump, which may be disposed of after use. In other embodiments the pump mechanism is only the unit comprising a pumping chamber and valves, and this mechanism is driven by an electric motor which is integrated in the durable unit. When the controlling unit is energized by its own electric cell it is avoided that data stored in this unit are deleted during the change of the disposable unit. It is possible to energize the controlling unit from the energy reservoir in the disposable unit so that only during change the energizing is switched over to the long life cell in the durable unit whereby this cell only acts as an emergency power supply.

The disposable unit may comprise sensors measuring the pressure in the catheter directly in the outlet from the pumping mechanism. The disposable unit may further comprise a memory keeping an account of the amount of liquid left in the reservoir. By enclosing such a memory in the disposable unit containing the liquid reservoir, this memory is firmly connected to the reservoir. This is appropriate if the memory shall be able to keep an account of the amount of liquid left in the reservoir. Data in the memory may be read out by the control unit and represented on the display.

The means for setting the controlling unit may comprise a socket with electrical contacts, which socket is designed to receive and communicate with a plug having corresponding electric contacts and carrying a programmed ROM-circuit defining the infusion data. The plug may further carry a graphic representation of the infusion data stored in the ROM, i.e. how the infusion of a set 24-hours' dose is distributed over the 24 hours. This graphic representation may be marks on a transparent sheet so secured to the plug that it covers a watch dial display when the plug is inserted in the socket, the marks indicating periods with increased or decreased infusion. By this construction the user may avoid the relatively complicated programming of the pump as he may plan the needed infusion profile or profiles in cooperation with his medical adviser and thereafter he will only have to insert the plug which is in accordance with his immediate life style.

In an attempt to make the infusion device even simpler in use, a number of fully disposable infusion devices has been proposed, see for example US 5 527 288 disclosing an intra-dermal drug delivery device which is entirely disposable and may be based either on mechanically or electronically controlled infusion means. Although this type of device provides for improved convenience and safety of use, it would normally be considered too costly to incorporate advanced functions such as flow measurement, display means or the ability to cooperate with a remote control unit. Addressing this problem, EP 1 177 802 describes an infusion device comprising a disposable pump and reservoir unit with an attachable durable portion, the latter comprising the control means for the pump.

As hollow from the above description of known devices, it is considered well known to "divide" an infusion device into a durable "control" unit and a disposable unit comprising a reservoir with a liquid to be infused, the actual mechanical, electrical and electronic interface between the two units being the result of a normal design procedure.

However, considering the infusion devices proposed during the last two decades, it becomes apparent that these devices are based upon, literally, "dividing" existing infusers into a durable portion comprising the more expensive components, and a disposable reservoir portion which is either inserted into a housing or attached to the durable portion in a click-on fashion. In other words, the developments made hitherto have entirely focused on the object of modifying existing concepts in order to improve the functionality experienced by the user.

This said, the present inventors have realised that the above concept of merely dividing what has hitherto been considered an infuser of a given type into a durable housing unit and a disposable cassette unit has lead to a number of restrictions, which restrictions can be divided into user-relevant restrictions and manufacturer-relevant restrictions.

From the point of the user, when acquiring a delivery device (typically an infusion pump device), it has to be decided which "type" of infuser the user considers to be the most appropriate at the given time. For example, a larger device comprising many features (e.g. programmability or a display) and which is adapted for larger cartridges/cassettes containing a larger amount of drug, such a device typically being relatively heavy and constructed to be suspended by a belt. Examples of this type of infusing device are disclosed in US 5 984 894 and FR-A-2 753 103. The user may also choose a smaller device which typically can be attached to the skin of the user by an adhesive, however, such infusion devices are normally rather small, with restricted capabilities in programmability. An example of this type of infusion device is disclosed in US 4 886 499.

In this way the user is "locked" with an infusing device which may not be the most appropriate under all circumstances, for example, under normal day to day conditions the user may prefer a larger device providing many user relevant features such as programmability, a display as well as a large reservoir. However, during sports exercise or when it is desirable to hide the infuser under light clothing, the appropriate infuser would be a small and simple device.

Indeed, it may be argued that it would be possible to attach a smaller reservoir unit to the larger type of infusion device, or to attach a larger reservoir unit to the smaller type of infusion device, however, this would hardly solve the problem as such infusion devices merely would be "a large infusion device with a small reservoir attached" or "a small infusion device with a large reservoir attached"; correspondingly, such infusion devices are not known in to the present inventors.

It may also be argued that infusion devices today have come down in price such that it would be within economical reach for the average user to have the desired number of different devices which would make it possible to just choose the type considered the most appropriate at a given time and for a given need. However, in practise this approach is not considered attractive by most users for a number of reasons, for example, most users prefer to get accustomed with only one type of infusion device, just as swapping between a number of different devices would make it difficult to keep track of the amount of drug infused. For these reasons, in practise, most users choose to live with the type of infusion device considered the most appropriate for their average needs.

From the point of the manufacturer, the practise hitherto pursued means that once it is decided to which "class" a given infusion device belongs, i.e. small or large, expensive or cheap, advanced or simple, it will be difficult and/or expensive to adopt a given device to suit other purposes. For example, it would be almost impossible to modify a "large-type" infusion device comprising pump and control means adapted for longevity, for example as known from FR-A-2 753 103, to provide a small and inexpensive device as known from US 4 886 499. Indeed, a given manufacturer may choose to develop two or more different infusion devices each belonging to a given class of devices as discussed above, however, this approach has at least two drawbacks. Firstly, it will be expensive to both develop and keep a number of systems technologically updated, and secondly, the user may choose different devices from different suppliers, this meaning loss of business as well as loss of brand loyalty.

### SUMMARY OF THE INVENTION

Having regard to the above discussion of known infusion devices, the object of the present invention is to provide a concept for a drug delivery device and system providing a high degree of flexibility for both the user and manufacturer, allowing the user to modify the infusion device or system to better suit the given and changing circumstances, and allowing the manufacturer to provide this flexibility in a cost effective and efficient manner.

The present invention is based on the realisation that the "type" or "class" for a given drug delivery device or system advantageously can be determined by an actual combination of units which allows a given number of inherently provided features (some or all) to be activated or made accessible depending on which units of a system is actually combined. Correspondingly, the invention provides a modular drug delivery system, comprising a reservoir and delivery unit and a control unit in combination with at least one further control unit, whereby each combination of a reservoir unit and a control provides different capabilities.

More specifically, in a general aspect the present invention provides a system for delivering a drug to a user, comprising a reservoir unit, a control unit, and at least one further control unit as defined in claim 1. The term "capabilities" is used to denote a set of actions or functions resulting from a combination of the two units. To provide the described functionality, the reservoir unit comprises receiving means for receiving the at least one command, and means for performing at least one action.

As appears, a plurality of different systems may be provided comprising different combinations of a reservoir unit and control units, a number of which will be described below.

In the context of the present application, a "command" provides or allows a given functionality, however, a command may typically comprise a series of signals providing a given action. For example, a command for a given delivery rate would normally be in the form of a series of signals actuating the expelling means thereby providing the desired action, e.g. a series of membrane strokes resulting in a given flow rate, or a series of signals controlling the contents of a display. Further, the nature of a command may be "active" such as when controlling a pump or a display, or "passive" when for example information is collected from a sensor element. Correspondingly, the term "command" may be said to represent a given function.

Further, the concept of controlling the operation of a local processor means includes both "active" and "passive" control of the commands. By active control is meant that a given command is activated, whereas passive control means that a given command is "allowed" to be expressed. For example, in case a number of delivery rate commands is provided, then a single command may be activated, whereas a command for controlling a display may always be "on", alone the presence of a display in the control unit will "control" the command. Also, a control processor may be used to control other elements than those controlled by a local processor, e.g. sensors.

The set of commands provided by the control means or the local processor means may comprise one or more commands controlling the expelling means. In a simple configuration the "set" of commands comprises a single command which would control the expelling means to expel drug contained in the reservoir corresponding to a pre-determined constant delivery rate, e.g. a basal rate, however, several such commands providing different delivery rates or profiles may be provided in combination with other commands. The expelling commands may also comprise one or more bolus commands by which a given volume of drug is infused over a shorter period.of time. In addition to commands controlling the expelling means numerous other commands may be provided, e.g. display commands may control display means, memory commands may control in- and output from memory means, sensor commands may control sensor means, alarm commands may control alarm means, and transmission commands may control the transmission of data information.

In the context of the present application, the term "different capabilities" is used to indicate that the "potential" capabilities are different, however, in case the capabilities are overlapping, the user may choose to operate a given control unit corresponding to the capabilities of another control unit, or two different advanced units may be used in the same way. For example, a control unit allowing user-programming of infusion profiles as well as a bolus function may be used to activate a simple constant flow rate corresponding to the capabilities of a simple control unit. This means that seen from the reservoir unit there will be no difference between the two control units.

According to the invention, the reservoir unit and at least one control unit comprise mating coupling means so as to allow the control unit to be secured to the disposable unit, the mating coupling means including communication means allowing information to be transferred, e.g. control commands and data information. The mating coupling means may include communication means comprising one or more of: electrical contacts means, opto-electrical means, wireless transmission means, or mechanical contact means allowing information to be transferred.

The mating coupling means may be adapted for releasably securing a control unit to a reservoir unit so as to allow the control unit to be readily removed and replaced when desired, or the mating coupling means may be adapted for permanently securing the control unit to the disposable unit.

The control or reservoir unit may be provided with more than one type of communication means, this allowing a given unit to communicate with different types of other units or to communicate with the same unit in different ways. The above-described "advanced" communication means may also be combined with means allowing for mechanical communication as will be described below. For example, a reservoir unit may be provided with first means which allows a simple mechanical control unit to start the infusion process, as well as second means allowing high-level bi-directional communication between the two units, either the same control unit or a different unit. A given control unit adapted for wireless communication may also be configured to be matingly attached to the reservoir unit in which case communication may still rely on wireless means or may be fully or partly replaced by contact means just as additional contact means by be used for transmission of additional commands or data.

In an exemplary embodiment at least one control unit comprises an attachable subunit and a remote subunit, the subunits comprising transmission means and corresponding receiving means adapted for wireless transmission of commands or data information to and/or from the respective unit(s). The reservoir unit and the attachable subunit comprise mating coupling means allowing the attachable subunit to be secured to the reservoir unit, the mating coupling means including communication means allowing commands or data information to be transferred, whereby commands or data information to and/or from the reservoir unit can be transmitted wireless between the remote subunit and the reservoir unit. By this configuration, the additional costs associated with the provision of wireless communication between the reservoir and a control unit can be transferred from the reservoir unit (which may be a prefilled disposable unit) to the durable control unit. A replaceable energy source may be provided in the attachable subunit for either energizing the delivery means in the reservoir unit or driving mechanical command means arranged in the attachable subunit. Indeed, for such a configuration the user will have to switch the attachable subunit each time the reservoir unit is changed, for which reason the assembled pump unit may be categorized as a semi-disposable system.

The energy source for driving the expelling means and the local processor may be supplied by a battery incorporated in the (disposable) reservoir unit, however, for environmental reasons the battery may be provided as a "reusable" unit which will have to be switched each time the reservoir unit is changed. In case a control unit is used which is fully or partly (e.g. as described above) attached to the reservoir unit during operation, energy may be supplied from this unit.

The outlet means associated with the reservoir may be.in direct fluid communication with the reservoir (e.g. in case the expelling means is arranged "before" the reservoir as for a piston pump) or indirect fluid communication (e.g. in case the expelling means is arranged "after" the reservoir as for a membrane pump). The outlet means may be adapted to be brought in fluid communication with infusion means (e.g. a catheter tubing or transcutaneous access means such as an infusion needle, a flexible infusion cannula or a plurality of micro-penetrators) or may comprise these. In the latter case the fluid communication may be established just prior to use, before or after the drug delivery device has been arranged on the user.

The delivery device may comprise outlet means being adapted to cooperate with or comprising infusion means. The infusion means may be in the form of a catheter tubing or transcutaneous access means such as an infusion needle, a flexible infusion cannula or a plurality of micro-penetrators. In exemplary embodiments the reservoir is a prefilled, flexible reservoir.

Display means may be provided on any of the units or subunits comprising a processor capable of driving a display, however, for cost reasons a display may be included only in a control unit or, alternatively, in one or both of its subunits. The display means may be used for displacing the contents of memory and/or setting means comprised in the delivery system.

A control may be provided with additional input and output means allowing it to communicate with external computer- and/or expert systems, just as the remote may be used in combination with a closed loop system comprising a blood glucose sensor.

In an exemplary embodiment the reservoir unit comprises a reservoir adapted to contain a liquid drug and comprising, in a situation of use, associated outlet means, expelling means for expelling a drug out of the reservoir through the outlet means, and local processor means providing a number of commands. One or more of the control unit comprises control means for controlling the operation of the local processor means, wherein each control unit is adapted to control a different command or combination of commands, thereby providing each combination of the disposable unit and a control unit with different capabilities. In this way the reservoir unit is provided as a unitary unit comprising both a reservoir and the expelling means.

In the context of the present invention, the term "capability" denotes an "activity", e.g. different reservoir units merely comprising different amounts or types of drug would not possess different capacities.

In a further exemplary embodiment the reservoir unit comprises a reservoir adapted to contain a liquid drug and comprising, in a situation of use, associated outlet means, the reservoir unit being adapted to receive at least one command from a control unit and perform an action in response thereto, the system comprising expelling means for expelling a drug out of the reservoir through the outlet means. Each control unit comprises control means providing one or more commands, in exemplary embodiments including at least one command controlling the expelling means, wherein each control unit is adapted to provide a different command or combination of commands, thereby providing each combination of the disposable unit and a control unit with different capabilities. The reservoir unit may comprise local processor means and at least one electronically controllable element associated with the local processor means, the local processor means being controllable by at least one control unit.

As appears, in contrast to the first described embodiment, the functionality of the reservoir unit may be fully controlled by the control unit, the reservoir unit merely providing actuatable means (e.g. expelling means such as a pump) controllable by commands from the control unit. In this context a "command" provides a given functionality and may be of either electrical or mechanical nature. For example, the reservoir unit may comprise a membrane pump being controlled directly by the control unit (i.e. the individual pump strokes), or the reservoir unit may comprise a pump of the constant flow-rate type (e.g. a bleeding hole pump) which is mechanically controlled by flow control means provided in the control unit. For example, in a very simple configuration, a bleeding hole pump comprises two different flow restrictors one of which is activated by a mechanical command when a corresponding control unit is locked in place. In this way a fully mechanical system may be provided comprising e.g. a spring driven pump and two mechanical control units by which one of two flow rates is selected when the corresponding control unit is attached.

The expelling means may be comprised in either the reservoir unit, the control unit or divided between the two units. In exemplary embodiments, the expelling means is fully comprised in the reservoir unit which is provided as a unitary unit. Depending on the nature of the expelling means, the expelling means *per se* may be fully comprised in the reservoir unit but with an interface controlled dynamically by the control unit. For example, a bleeding hole pump may be activated by a mechanical "on" command which opens for the flow of drug, and subsequently controlled by a "flow rate" command in which a flow conduit is opened and closed by means arranged in the control unit.

In exemplary embodiments, the expelling means is controlled.by a control unit providing at least one command controlling the expelling means, the control unit being adapted to control at least one command controlling the expelling means (either directly or via local processor means), however, the expelling means may be controlled independently of the control unit. For example, a reservoir unit may be adapted to provide a given delivery rate without the cooperation of a control unit, the delivery means being either "self-controlled" (e.g. a spring-driven bleeding hole pump or an osmotic pump) or electronically controlled by local processor means (e.g. a membrane or piston pump). In this configuration the control units merely provide additional functions by controlling additional commands or actions, e.g. transmission commands, display commands, memory commands etc.

In a further aspect the present invention provides a system for delivering a drug to a user, comprising at least first and second reservoir units and at least first and second control units.

In an exemplary embodiment at least the first and second reservoir units each comprises a reservoir adapted to contain a liquid drug and comprising, in a situation of use, associated outlet means, expelling means for expelling a drug out of the reservoir through the outlet means, local processor means providing one or more commands, wherein each reservoir unit provides a different combination of commands. At least the first and second reservoir units each comprises control means for controlling the operation of a local processor means, wherein the control means is adapted to control different commands or, at least partially, different combinations of commands, thereby providing each combination of a reservoir unit and a control unit with different capabilities.

In a further exemplary embodiment each reservoir unit comprises a reservoir adapted to contain a liquid drug and comprising, in a situation of use, associated outlet means, each reservoir unit being adapted to receive at least one command from the control unit and perform an action in response thereto, the system comprising expelling means for expelling a drug out of the reservoir through the outlet means. Each control unit comprises control means providing one or more commands, wherein each control unit is adapted to provide a different command or combination of commands, thereby providing each combination of a reservoir unit and a control unit with different capabilities.

In the above disclosure of the invention, the components of the system have been described primarily with respect to technical features. In the hollowing different exemplary "user-oriented" configurations will be disclosed.

In one aspect, the "external" configuration of the present invention may be based on the concept of a reservoir unit (e.g. a disposable, prefilled unit) defining the outer boundaries, or the platform, for the system to which different control units (e.g. durable units) can be attached, the different control units providing combined devices with different capabilities, this in contrast to most of the known systems which are based on the concept of the durable unit defining, in general, the platform and the outer boundaries for the combined infusion system, the disposable reservoir unit being inserted or attached to this platform.

When the control unit is attached to the reservoir during operation, the resulting drug delivery device may be considered a "combined" device comprising a reservoir unit arid a control unit. The combined device comprises expelling means (e.g. a pump) for expelling a liquid from the reservoir, however, according to the specific design thereof, the expelling means may be comprised either entirely in the reservoir unit or it may be made up of a liquid-contacting portion comprised in the reservoir unit and adapted to cooperate with expelling means comprised in the control unit.

The control unit may be attached in any desirable way. In an exemplary configuration the control unit is inserted into an opening or recess of the reservoir unit adapted to accommodate and connect to the control unit, the units comprising corresponding mating coupling means. For such a configuration the reservoir unit could be said to represent a housing into which the control unit is inserted fully or partly, i.e either flush with a surface of the disposable unit or protruding more or less. In another exemplary embodiment the control unit is in the form of a member which can be attached onto the housing of the disposable unit, for example along an edge thereof. In case the control unit is in the form of remote control unit, the units may be adapted to be attached to each other in the same way when it is desirable to have only one unit.

In an exemplary embodiment the reservoir unit comprises a housing having a first surface configured to be arranged against the skin surface of a user, and a second surface facing away from the first surface and comprising means for allowing a control unit to be attached in a mating relationship. In an exemplary embodiment the first surface is provided with an adhesive means for attaching the housing to the skin of the user, the second surface preferably being arranged substantially opposite the first surface.

As discussed above, the actual mechanical, electrical and electronic interface between the two units is decided during a normal design procedure, however, the interface should designed in such a way, that the highest degree of constructional freedom is provided for both of the units, allowing the "controlling capabilities" of the control unit to be varied from very complex versions to very simple versions.

More specifically, the control unit may comprise control means adapted primarily to provide a user interface allowing the user to "communicate" with the infusion device, e.g. providing the user with information of the present settings, the amount of previously infused drug for a, given period, the type of drug in the actual disposable unit and the amount remaining, as well as allowing the user to program or change the actual settings. The control unit may comprise an energy source for providing the control means with electric energy, this allowing the control unit to be operated when disconnected from the reservoir unit as well as providing energy for supporting any volatile information stored in the control unit (e.g. individual settings and information of infusion history), however, energy for operating the control unit when attached to the reservoir unit may be provided from an energy source within the latter.

The interface between the control and the reservoir unit is advantageously designed for communication between different "types" of means for a given functionality such that a general set of control commands (e.g. for a given infusion rate) can be used to control a diversity of expelling means having diverse nature. In the same way, different types of reservoir units may communicate with the control means also using a general set of commands informing the control means about the type of disposable unit, the amount remaining the reservoir etc.

The control or the reservoir unit may be provided with more than one type of communication means, this allowing a given unit to communicate with different types of other units. The above-described "advanced" communication means may also be combined with means allowing for mechanical communication as will be described in greater detail below. For example, a reservoir unit may be provided with first means which allows a simple mechanical control unit to start the infusion process, as well as second means allowing high-level bi-directional communication between the two units.

In the above, relatively complex control units have been described having advanced control or display features which in most cases require the provision of electronic means to be implemented. However, it is also within the scope of the present invention to provide much simpler control units based for example on "mechanical communication" between the control unit and the reservoir unit. In a very simple form the control unit functions merely as an "on-button" when attached, or as an "on-off" switch in case the control unit is detachable. Such a simple control unit may further comprise means allowing the disposable unit to be "programmed" (i.e. activating a given set of commands) depending on the configuration of the control unit. For example, the reservoir unit may be configured to provide a plurality of different basal rates, the actual basal rate being set by the configuration of the control unit. For this kind of control different control units may be provided for each basal rate or a single control unit may be set in accordance with the desired rate (e.g. in accordance with the orientation when attached). In exemplary embodiments of the above-described simple versions of the control unit, the communication between the control unit and the reservoir unit is based on mechanical interactions; whereby the mechanical configuration of the control unit is detected by the reservoir unit. The mechanical control means may cooperate with any suitable kind of contact means in the reservoir unit, e.g. electrical contacts or opto-electrical means.

Although the above-described exemplary embodiments provide a large degree of constructional freedom, it is also within the scope of the present invention to provide cooperating units which to a higher degree is adapted specifically to each other. For example, the control unit may comprise pump means in the form of a peristaltic pump which will only operate in combination with a reservoir unit comprising expelling means in the form of a corresponding tubing, or a drive means adapted to operate a valve-controlled expelling pump associated with the reservoir.

In the above description of the invention the terms "disposable" and "durable" have been used, the latter traditionally defining the part of the system which as a consequence of a relatively high price is intended for being used for a longer period, normally several years. Typically, the high price was due to the cost of a relatively expensive housing as well as pump and control means, the latter including programming means. However, strictly speaking, any "durable" unit comprising the above components may be considered a disposable unit in case the cost thereof is sufficiently low. In the same way, the "disposable" unit may be semi-disposable such that the reservoir can be re-filled with a given drug.

By (also) providing a disposable control unit a number of advantages is achieved from the sight of the manufacturer as well by allowing a large number of variants to be produced and distributed in an effective manner. For example, different versions of the reservoir unit as well as different versions of the control unit may be produced at different locations and shipped independently of each other to a given location, where the units may be assembled, for example at the point of sale. In this way cheap or expensive versions of the two units may be combined as desired. When the control unit is sold as a disposable component, the mating coupling means may be adapted to permanently secure the control unit to the housing preventing the reuse of the control unit. Actuating the system to start drug delivery may be by further manipulation of the control unit (e.g. pressing or turning) or by additional means provided on the reservoir unit. In this way it would be possible to produce one or more control units which just prior to installation are given a functionality corresponding to either a cheap or a more expensive version.

In exemplary embodiments, the coupling means provided in the reservoir unit is of a "general" type allowing different control modules to be locked in either a permanent or a releasable manner in accordance with the type of control module.

The expelling means may be of any desirable nature, such as known from US patents 4,340,048 and 4,552,561 (based on osmotic pumps), US patent 5,858,001 (based on a piston pump), US patent 6,280,148 (based on a membrane pump), US patent 5,957,895 (based on a flow restrictor pump (also know as a bleeding hole pump)), or US patent 5,527,288 (based on a gas generating pump), which all in the last decades have been proposed for use in inexpensive, primarily disposable drug infusion pumps; the cited documents being incorporated by reference.

The outlet means associated with the reservoir may be in direct fluid communication with the reservoir (e.g. in case the expelling means is arranged "before" the reservoir as for a piston pump) or indirect fluid communication (e.g. in case the expelling means is arranged "after" the reservoir as for a membrane pump). The outlet means may be adapted to be brought in fluid communication with infusion means (e.g. a catheter tubing or transcutaneous access means such as an infusion needle, a flexible infusion cannula or a plurality of micro-penetrators) or may comprise these. In the latter case the fluid communication may be established just prior to use, before or after the drug delivery device has been arranged on the user.

The reservoir may be prefilled with the liquid to be infused and thus ready to be used, or it may be adapted to be filled with the liquid from an external source prior to use, the latter in case a prefilled reservoir will result in reduced shelf-life for the contained liquid.

As used herein, the term "drug" is meant to encompass any drug-containing flowable medicine capable of being passed through a delivery means such as a hollow needle in a controlled manner, such as a liquid, solution, gel or fine suspension. Representative drugs include pharmaceuticals such as peptides, proteins, and hormones, biologically derived or active agents, hormonal and gene based agents, nutritional formulas and other substances in both solid (dispensed) or liquid form. In the description of the exemplary embodiments reference will be made to the use of insulin. Correspondingly, the term "subcutaneous" infusion is meant to encompass any method of parenteral delivery to a subject.

### BRIEF DESCRIPTION OF THE DRAWINGS

In the following the invention will be further described with references to the drawings, wherein
fig. 1 is a schematic representation of a first embodiment of the invention,
figs. 2A-2B show a reservoir unit useful with the present invention,
figs. 3A-3B show a further reservoir unit useful with the present invention,
figs. 4A-4C show a further reservoir unit useful with the present invention.

In the figures like numerals are used to denote like or similar structures.

### DESCRIPTION OF EXEMPLARY EMBODIMENTS

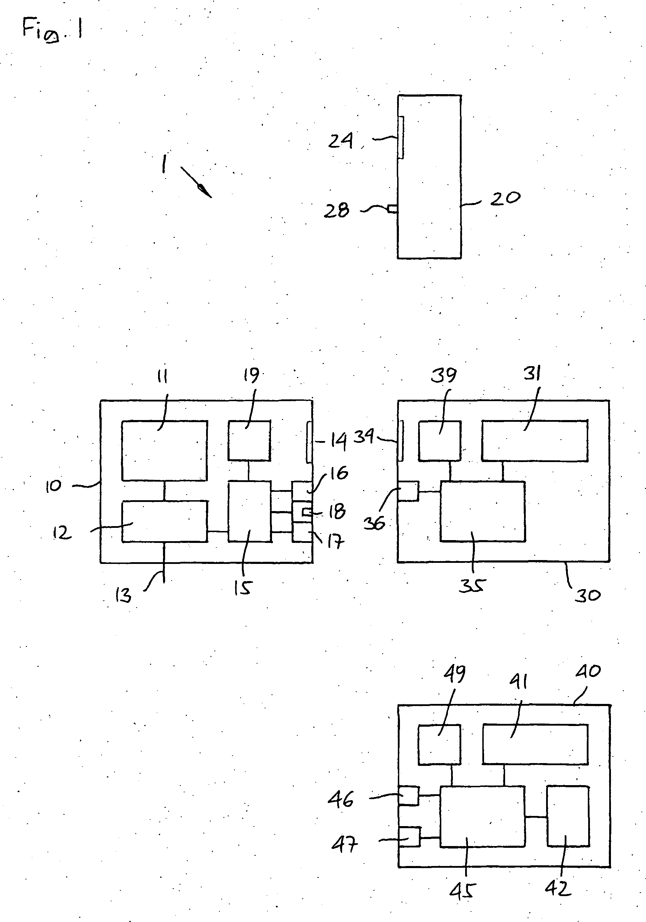

Fig. 1 shows a schematic representation of a first embodiment of the invention. Correspondingly, the configuration of the different structures as well as there relative dimensions and locations are intended to serve illustrative purposes only.

More specifically, a drug delivery system (here: infusion system) comprises a reservoir unit 10 in combination with three different control units 20, 30, 40, in which a combination of the reservoir unit and one of the control units provides an operative drug delivery device.

The reservoir unit comprises a drug reservoir 11 and a pump 12 comprising an outlet means 13 and adapted for infusing a drug into a body of a user in accordance with instructions (i.e. a local command) received from a local processor 15. The pump may be of the metering type, i.e. the amount of drug infused corresponds to the controlling signals received from the local processor or the infusion unit may be provided with detecting means for determining the amount of drug actually infused (not shown). The local processor is associated with a local receiving means 16 cooperating with the local processor means for receiving control commands from at least one of the control units. An energy source 19 is provided in the form of a battery.

The three control units are in the form of a basic unit 20, a standard unit 30 and an advanced unit 40. The standard control unit comprises a control processor 35 associated with a transmitter 36 for wireless transmitting control commands to the local processor via the local receiving means. The control unit further comprises a display 31 associated with the control processor. The display may be used when the user enters information into the control unit via user-accessible input means (not shown), e.g. the desired size for a bolus command or program data changing the infusion rate or profile, the information being transferred via the transmitting means. An energy source 39 is provided in the form of a battery. The standard control unit 30 may be attached to the reservoir unit 10 by releasable means 14, 34.

The first combination is based on one-way transmission of commands from the control unit to the reservoir unit, however, the reservoir unit comprises local transmission means 17 cooperating with the local processor means for transmitting data information to control receiving means 47 provided in the advanced control unit 40. The data may comprise information as to the initial amount of drug in the reservoir, the current amount of drug in the reservoir, the infusion rate, information from a sensor element or ID information identifying the given reservoir unit. The advanced control unit further comprises memory means 42 allowing transmitted and/or received commands/data to be stored and recalled. The memory means may be detachable, e.g. in the form of a memory stick or card.

The first and second combinations are based on one-way transmission of commands from the control unit to the reservoir unit, however, the reservoir unit further comprises second local receiving means 18 adapted to cooperate mechanical command means 28 provided on the basic control unit 20. More specifically, the basic control unit is provided with a protrusion 28 adapted to be received in the second local receiving means when the reservoir unit and the control unit are attached to each other by releasable or non-releasable means 14, 24. The reservoir unit may be adapted to provide a single basal infusion rate when combined with the basic control unit, or it may be adapted to provide a plurality of infusion rates which can be specifically activated by mechanical commands from a corresponding number of different basic control units.

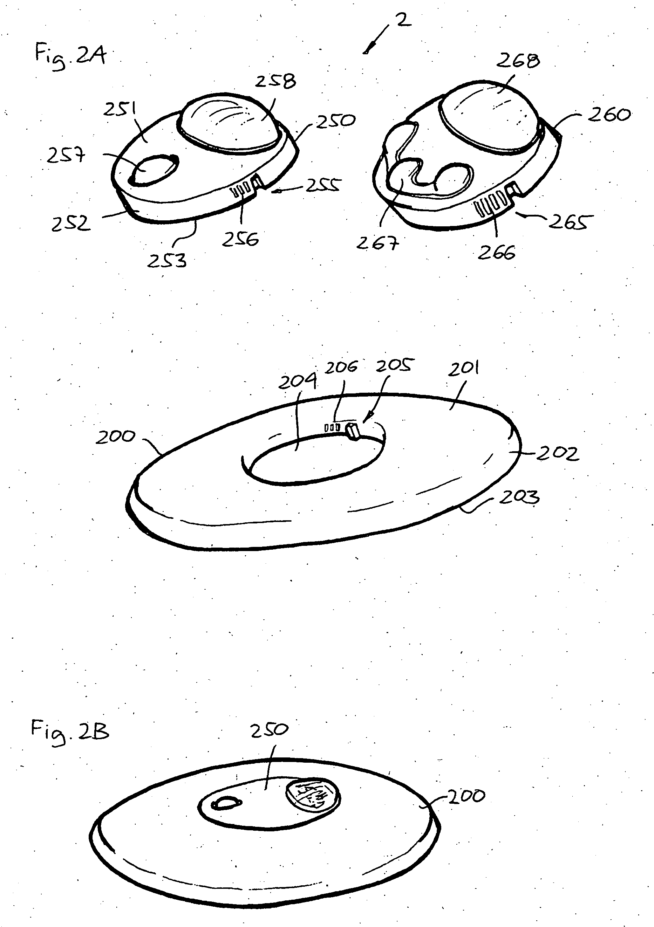

Figs. 2A and 2B show a second embodiment of the invention. More specifically, a drug delivery (here: infusion) system 2 comprises a reservoir unit 200 in combination with two different control units 250, 260, in which a combination of the reservoir unit and one of the control units provides an operative drug delivery device.

The reservoir unit comprises an outer housing defining the outer boundaries for the combined infusion device, the housing having an upper surface 201, a rim portion 202 and a lower surface 203 adapted to be arranged towards a skin surface of a user. Preferably the lower surface is provided with an adhesive (not shown) allowing the device to be attached directly to the skin surface. In the upper surface an opening 204 is provided adapted to receive a control unit, the reservoir unit and the control units including mating coupling means 205, 255, 265 so as to allow a control unit to be secured to the reservoir unit, the mating coupling means including electrical contacts 206, 256, 266.

The control unit 250 comprises a housing having an upper surface 251, a rim portion 252 and a lower surface 253, the control unit being adapted to be received in a corresponding opening in the reservoir unit 200, the control unit including mating coupling means 255 so as to allow the control unit to be secured to the disposable unit. It is to be noted that the coupling means shown on the control units respectively the reservoir unit are not intended to engage each other but are merely arranged for illustrative purposes. In accordance with the actual design of the units, the electrical contacts may provide command/data transfer to and/or from the control unit as well as providing the control unit with energy from an energy source provided in the disposable unit or vice versa.

The control unit 250 comprises a user interface having a user actuatable key 257 and a display 258. In the shown embodiment only a single key is provided, for example in the form of an on-off switch or a bolus key. In accordance with the actual design of the control unit, the display may provide the user with different kinds of information, e.g. the actual infusion profile, the amount of drug infused (e.g. for insulin a number of units) for a given period, the amount of drug remaining in the disposable unit etc.

As the basal infusion rate for any drug to be infused using the device of the invention will be dependent of the individual user, it could be possible to set such a basal rate using control means arranged on either of the two units. As this setting normally will not have to be changed on a regular basis, these control means may be provided on portions of the units which are hidden to the user when the two units are secured to each other, for example on the rear surface of the control module.

The second control unit 260 essentially corresponds to the first control unit 250, the differences being a group of user actuatable keys 267, e.g. allowing the unit to be programmed by the user, as well as another display 268, e.g. having graphic and/or alpha-numeric capabilities. The second control unit further comprises a memory means as well as a back-up energy source therefore (not shown). In the shown embodiment all pump components in contact with the drug is contained in the disposable part, however, according to the actual type of pump, some components thereof may be provided in the control unit, for example a small electric motor actuating a valve pump comprised in the disposable unit. The memory means allows the control unit to be released from a first disposable unit and placed in a second disposable unit without loss of information contained in the control unit, e.g. specific program settings or information regarding the amount of drug infused for one or more periods.

In a further embodiment (not shown), the disposable unit may comprise additional means providing a back-up memory function for the control unit, this wallowing a new or pre-used control unit to be updated when inserted into such a disposable unit.

Fig. 2B shows the reservoir unit 200 with the control unit 250 mounted. As can be seen, the control unit is fully embedded in the opening of the reservoir unit, the upper surfaces of the two units being arranged flush with each other. Advantageously, such a design could be used in cases where the combined device is sold as a disposable single unit (preferably with a basic control unit as described above), the control unit being inserted into the opening before being sold. Indeed, the shown flush configuration may also be used for control units intended for re-use.

Not to be seen, the reservoir unit comprises a reservoir, a pump means for pumping drug contained in the reservoir to an outlet opening (not shown) and an energy source for operating the pump and, if deemed appropriate, the control unit when attached. As described above with reference to the second control unit 260, the pump means may serve fully or only partly as an operating pump, the remaining components being provided by the control unit. Further, depending on the actual type and design of the pump, control means for operating the pump may be provided in either of the units or divided therebetween as discussed in greater detail in the introductory portion.

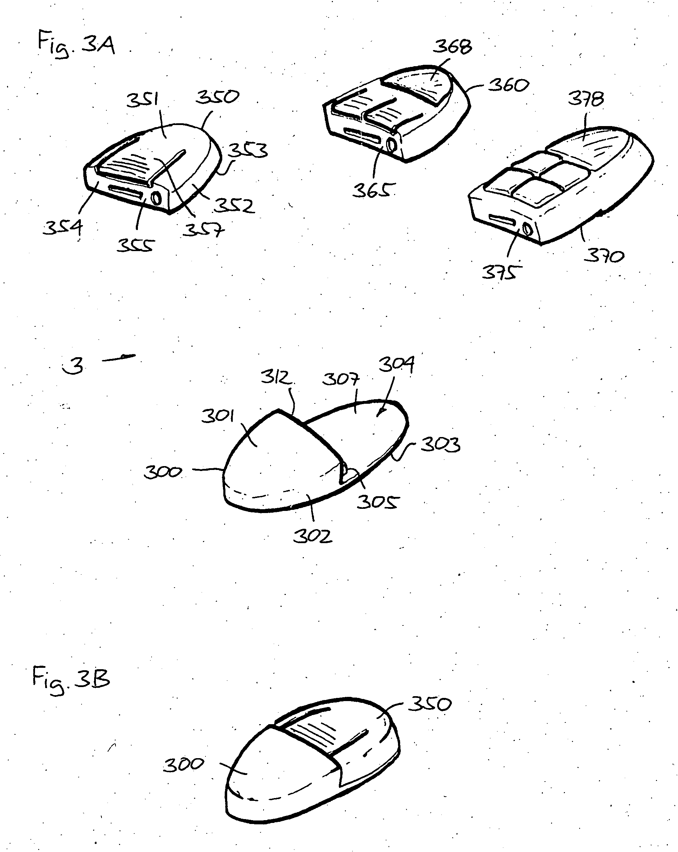

Figs. 3A and 3B show a third embodiment of the invention. More specifically, a drug delivery (here: infusion) system 3 comprises a reservoir unit 300 in combination with three different control units 350, 360, 370 in which a combination of the reservoir unit and one of the control units provides an operative drug delivery device.

The reservoir unit comprises an outer housing defining the outer boundaries for the combined infusion device, the housing having an upper surface 301, a rim portion 302 and a lower surface 303 adapted to be arranged towards a skin surface of a user and preferably being provided with an adhesive. The reservoir unit is provided with a "cut-out" portion 304 adapted to receive a control unit, the reservoir unit and the control units including mating coupling means 305, 355, 365, 375 so as to allow the control unit to be secured to the reservoir unit, the mating coupling means including electrical contacts. As can be seen, the lower portion comprises an extension in the form of a base plate portion which extends in a tongue-like fashion corresponding to the cut-out portion of the housing, the upper surface 307 of the base plate portion and the portion 312 of the rim facing towards the base plate portion providing mounting surfaces for a control unit and together defining a confinement for a control unit. As appears from the figure, the confinement 304 is provided within the outer boundaries of the disposable housing. The reservoir unit 300 comprises the same internal components as described for the second embodiment.

The control unit 350 comprises a housing having an upper surface 351, a rim 352 and a lower surface 353, the rim comprising a connecting portion 354. The control unit is adapted to be received in the corresponding confinement 304 in the reservoir unit 300, the control unit including mating coupling means 355 on the connecting portion 354 and the lower surface (not shown) so as to allow the control unit to be secured to the corresponding portions 312, 307 of the reservoir unit, the mating coupling means including electrical contacts. The coupling means may be of the same type as discussed above with reference to the fig. 2A embodiment. The control unit 350 comprises a simple user interface having a single user actuatable key 357, for example in the form of an on-off key or a bolus key, but no display.

The second control unit 360 has the same dimensions as the first control unit 350, but comprising two keys and a display 368. The display may provide the user with information as to infusion settings and/or history, just as the display would allow the control unit to be menu-programmable using the two keys. The third control unit is somewhat larger (and thus larger than the upper base plate surface 307) and comprises four keys and a larger display 378.

Fig. 3B shows the reservoir unit 300 with the control unit 350 mounted As can be seen, the control unit fully occupy the confinement 304 defined within the outer boundaries of the disposable housing, the upper and outer rim surfaces of the two units being arranged flush with each other. The control unit may be attached in releasable or a non-releasable manner.

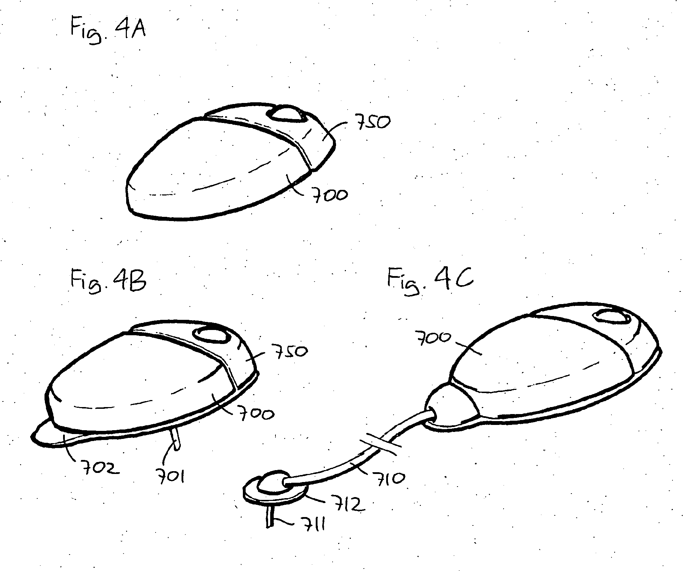

Fig. 4A discloses a control unit 750 mounted in a reservoir unit 700. Apart from the outer form of the control unit and the corresponding confinement in the reservoir unit, the units are of the same general configuration as the embodiment shown in fig. 3B to which reference is made. Fig. 4B shows the combined infusion device of fig. 4A with a hollow infusion needle 701 attached in communication with an outlet from the pump or reservoir means and protruding from the lower surface of the reservoir unit. The lower surface is further provided with a peelable liner 702 covering an adhesive layer for attaching the device to the skin of the user.
To facilitate introduction of the needle, the disposable device may be provided with user actuatable means for automatically advancing the needle into the skin after the device has been attached. Fig. 4C shows the infusion device of fig. 4B with an infusion catheter attached in communication with an outlet from the pump means. The infusion catheter comprises an infusion line or tubing 710 communicating with an infusion needle 711, a pad 712 being provided between the line and the needle, the pad allowing the catheter and needle to be gripped during the insertion procedure just as it allows the catheter to be properly secured to the skin of the user.

The reservoir unit may be provided with an outlet opening in the vicinity of the lower surface thereof comprising a connector allowing either an infusion needle or an infusion catheter to be attached to the opening. Such a connector may also serve as a port for filling the reservoir, either initially or during re-filling.

While the present invention has been described in connection with the exemplary embodiments shown in the various figures, the delivery system according to the invention may be provided with additional features providing improved functionality, control and ease of use.

For example, a sensor may be provided for continuously measuring the pressure in the infusion line or needle, this allowing the detection of a malfunctioning which could be used to initiate an alarm making the user aware of a problem.

However, more advanced sensors may be incorporated in the units. For example, it may be desirable to automatically deliver certain drugs only when required by the subject, either by patient activation or passively, such as by a feedback mechanism. In such a case, the device further includes a sensor (feedback) for detecting a condition in the body of the subject and for controlling the delivery of the drug in response thereto. The sensor may be, for example, a temperature sensor, a pulse rate sensor, a blood glucose sensor, a blood pressure sensor or a pH sensor. The sensors may be formed integrally with the reservoir unit or attached separately. The sensor may rest against the skin, may be inserted through the skin, or may be within the device and separate from the skin.

The reservoir unit may also include a plurality of drug reservoirs, each reservoir being independently controllable and communicating with an outlet with which a single infusion needle also communicates. For such a plurality of drug reservoirs, pump means integrally formed with the reservoir is preferred, for example in the form of individual gas generators. Including a plurality of drug reservoirs provides for considerable variations in the amounts of drug which can be delivered, in the rates at which drug can be delivered and in the number of drugs which can be delivered by the same device. The reservoir unit may further be provided with a reservoir for a calibrating liquid in case the unit is provided with sensors requiring such a liquid.

In the above description of the exemplary embodiments, the different structures providing mechanical, electrical and fluid contact and communication between the different components just as the means providing the described functionality for the different components (i.e. pump, reservoir, energy source, memory, control, display etc.) have been described to a degree to which the concept of the present invention will be apparent to the skilled reader. The detailed construction and specification for the different components are considered the object of a normal design procedure performed by the skilled person along the lines set out in the present specification.

## Claims

1. A modular system (1) for delivering a drug to a user, comprising:
- a first control unit (20) comprising a control means providing one or more control commands, and
- a reservoir unit (10) comprising a reservoir (11) adapted to contain a liquid drug and comprising, in a situation of use, associated outlet means (13), and receiving means (16, 17, 18) adapted to receive at least one control command from the first control unit and perform an action in response thereto,
- wherein the reservoir unit and the control unit comprise mating coupling means (14, 24) allowing a control unit to be secured to the reservoir unit to thereby provide a combination thereof, the combination comprising expelling means (12) for expelling a drug out of the reservoir through the outlet means, and an adhesive for attaching the combination to the skin of the user,
- at least one further control unit (30), each further control unit comprising a control means providing one or more control commands, and mating coupling means (34) allowing each further control unit to be secured to the reservoir unit,
- wherein the control commands include at least one control command controlling the delivery rate or delivery profile for the expelling means, wherein each control unit is adapted to provide a different control command, thereby providing each combination of the reservoir unit and a control unit with different capabilities in respect of the delivery rate or delivery profile of the expelling means, **characterized in that**
- the control means has a mechanical configuration providing the control commands for controlling the delivery rate or delivery profile for the expelling means, and
- the reservoir unit comprises contact means adapted to cooperate with the mechanical control means to detect the mechanical configuration thereof.

2. A system as defined in claim 1, wherein the reservoir unit comprises:
- the expelling means (12),
- local processor means (15) providing a number of local commands,
- means for performing at least one action in response to a local command,
wherein the at least first and second control units each comprise control means (35, 45) providing one or more control commands for controlling the operation of the local processor means, wherein each control unit is adapted to control a different local command or combination of local commands.

3. A system as defined in claim 2, wherein the reservoir unit further comprises at least one electronically controllable element (12) associated with the local processor means, the local processor means being controllable by at least one control unit.

4. A system as defined in claims 2 or 3, wherein the reservoir unit comprises:
- local receiving means (16) cooperating with the local processor means (15) for receiving control commands from at least one control unit,
- at least one of the control units comprising:
- control processor means (35), and
- control transmission means (36) cooperating with the control processor means for transmitting control commands to the local receiving means for controlling a local command or a combination of local commands.

5. A system as defined in any of claims 2-4, wherein the reservoir unit comprises:
- local transmission means (17) cooperating with the local processor means (15) for transmitting data information to control receiving means (35) in at least one control unit,
- at least one of the control units comprising:
- control receiving means (47) cooperating with control processor means (45) for receiving data information from the local processor.

6. A system as defined in claim 4 or 5, wherein at least one transmission means (16) and the corresponding receiving means (36) are adapted for wireless transmission of commands or data information.

7. A system as defined in any of claims 2-6, wherein the mating coupling means includes communication means (18, 28) allowing information to be transferred.

8. A system as defined in any of claims 1-7, comprising at least one further reservoir unit.

9. A system as defined in any of the previous claims, wherein at least one unit further comprises display means (268) associated with and controlled by processor means (145).

10. A system as defined in any of the previous claims, wherein at least one reservoir unit is in the form of a disposable, prefilled unit.

11. A system as defined in any of the previous claims, wherein at least one reservoir unit comprises a sensor, preferably associated with a local processor means.

12. A system as defined in any of the previous claims, wherein at least one reservoir unit comprises a surface (203) configured to be arranged against a skin surface of a user.

13. A system as defined in any of the previous claims, further comprising a sensor for continuously measuring a pressure associated with the outlet means.

14. A system as defined in any of the previous claims, further comprising a sensor for detecting a condition in the body of the user, and means for controlling the delivery of the drug in response thereto.

15. A system as defined in any of the previous claims, wherein at least one control unit comprises memory means.

## Patentansprüche

1. Baukastensystem (1) zum Abgeben eines Arzneistoffs an einen Anwender, umfassend
- eine erste Steuereinheit (20), umfassend ein Steuermittel, das einen oder mehrere Steuerbefehle bereitstellt, und
- eine Behältereinheit (10), umfassend einen zur Aufnahme eines flüssigen Arzneistoffs angepassten Behälter (11) und ein in einer Verwendungssituation damit verbundenes Auslassmittel (13) und Empfangsmittel (16, 17, 18), das dazu angepasst sind, mindestens einen Steuerbefehl von der ersten Steuereinheit zu empfangen und als Reaktion darauf eine Handlung auszuführen,
- wobei die Behältereinheit und die Steuereinheit ein Kupplungsmittelpaar (14, 24) umfassen, durch welches sich eine Steuereinheit an der Behältereinheit befestigen lässt, um **dadurch** eine Kombination davon bereitzustellen, wobei die Kombination ein Ausstoßmittel (12) zum Ausstoßen eines Arzneistoffs aus dem Behälter durch das Auslassmittel und einen Klebstoff zum Anbringen der Kombination an der Haut des Anwenders umfasst,
- mindestens eine weitere Steuereinheit (30), wobei jede weitere Steuereinheit ein Steuermittel, das einen oder mehrere Steuerbefehle bereitstellt, und ein Kupplungsmittelpaar (34), durch welches sich jede weitere Steuereinheit an der Behältereinheit befestigen lässt, umfasst,
- wobei die Steuerbefehle mindestens einen Steuerbefehl einschließt, der die Abgabegeschwindigkeit oder das Abgabeprofil für das Ausstoßmittel steuert, wobei jede Steuereinheit dazu angepasst ist, einen unterschiedlichen Steuerbefehl bereitzustellen, wodurch jede Kombination aus der Behältereinheit und einer Steuereinheit mit unterschiedlichen Fähigkeiten in Bezug auf die Abgabegeschwindigkeit oder das Abgabeprofil des Ausstoßmittels versehen wird, **dadurch gekennzeichnet, dass**
- das Steuermittel einen mechanischen Aufbau aufweist, der die Steuerbefehle zum Steuern der Abgabegeschwindigkeit oder des Abgabeprofils für das Ausstoßmittel bereitstellt, und
- die Behältereinheit ein Kontaktmittel umfasst, das dazu angepasst ist, mit dem mechanischen Steuermittel derart zusammenzuarbeiten, dass dessen mechanischer Aufbau ermittelt wird.

2. System wie in Anspruch 1 definiert, wobei die Behältereinheit Folgendes umfasst:
- das Ausstoßmittel (12),
- ein lokales Prozessormittel (15), das eine Anzahl an lokalen Befehlen bereitstellt,
- ein Mittel zum Ausführen mindestens einer Handlung als Reaktion auf einen lokalen Befehl,
wobei die mindestens erste und zweite Steuereinheit jeweils ein Steuermittel (35, 45) umfassen, die zum Steuern des Betriebs der lokalen Prozessoreinheit einen oder mehrere Steuerbefehle bereitstellen, wobei jede Steuereinheit dazu angepasst ist, einen unterschiedlichen lokalen Befehl oder eine unterschiedliche Kombination von lokalen Befehlen auszuführen.

3. System wie in Anspruch 2 definiert, wobei die Behältereinheit ferner mindestens ein elektronisch steuerbares Element (12) umfasst, das mit dem lokalen Prozessormittel verbunden ist, wobei das lokale Prozessormittel durch mindestens eine Steuereinheit steuerbar ist.

4. System wie in den Ansprüchen 2 oder 3 definiert, wobei die Behältereinheit Folgendes umfasst:
- ein lokales Empfangsmittel (16), das mit dem lokalen Prozessormittel (15) derart zusammenarbeitet, dass von mindestens einer Steuereinheit Steuerbefehle empfangen werden,
- wobei mindestens eine der Steuereinheiten Folgendes umfasst:
- ein Steuerprozessormittel (35) und
- ein Steuerübertragungsmittel (36), das mit dem Steuerprozessormittel derart zusammenarbeitet, dass Steuerbefehle zu dem lokalen Empfangsmittel übertragen werden, um einen lokalen Befehl oder eine Kombination von lokalen Befehlen zu steuern.

5. System wie in einem der Ansprüche 2-4 definiert, wobei die Behältereinheit Folgendes umfasst:
- ein lokales Übertragungsmittel (17), das mit dem lokalen Steuerprozessormittel (15) derart zusammenarbeitet, dass Dateninformationen an das Steuerempfangsmittel (35) in mindestens einer Steuereinheit übertragen werden,
- wobei mindestens eine der Steuereinheiten Folgendes umfasst:
- ein Steuerempfangsmittel (47), das mit dem Steuerprozessormittel (45) derart zusammenarbeitet, dass Dateninformationen vom lokalen Prozessor empfangen werden.

6. System wie in Anspruch 4 oder 5 definiert, wobei mindestens ein Übertragungsmittel (16) und das entsprechende Empfangsmittel (36) zur drahtlosen Übertragung von Befehlen oder Dateninformationen angepasst sind.

7. System wie in einem der Ansprüche 2-6 definiert, wobei das Kupplungsmittelpaar ein Kommunikationsmittel (18, 28) einschließt, das die Übertragung von Informationen ermöglicht.

8. System wie in einem der Ansprüche 1-7 definiert, das mindestens eine weitere Behältereinheit umfasst.

9. System wie in einem der vorangehenden Ansprüche definiert, wobei mindestens eine Einheit ferner ein Anzeigemittel (268) umfasst, das mit dem Prozessormittel (145) verbunden ist und **dadurch** gesteuert wird.

10. System wie in einem der vorangehenden Ansprüche definiert, wobei mindestens eine Behältereinheit in der Form einer vorgefüllten Einwegeinheit vorliegt.

11. System wie in einem der vorangehenden Ansprüche definiert, wobei mindestens eine Behältereinheit einen Sensor umfasst, der vorzugsweise mit dem lokalen Prozessormittel verbunden ist.

12. System wie in einem der vorangehenden Ansprüche definiert, wobei mindestens eine Behältereinheit eine Oberfläche (203) umfasst, die derart konfiguriert ist, dass sie an der Hautoberfläche eines Anwenders angeordnet werden kann.

13. System wie in einem der vorangehenden Ansprüche definiert, ferner umfassend einen Sensor zum kontinuierlichen Messen eines mit dem Auslassmittel verbundenen Drucks.

14. System wie in einem der vorangehenden Ansprüche definiert, ferner umfassend einen Sensor zum Ermitteln eines Zustands im Körper des Anwenders und ein Mittel zum Steuern der Abgabe des Arzneistoffs als Reaktion darauf.

15. System wie in einem der vorangehenden Ansprüche definiert, wobei mindestens eine Steuereinheit ein Speichermittel umfasst.

## Revendications

1. Un système modulaire (1) pour délivrer un médicament à un utilisateur, comprenant :
- une première unité de contrôle (20) comprenant des moyens de contrôle produisant une ou plusieurs commandes de contrôle, et
- une unité réservoir (10) comprenant un réservoir (11) adapté pour contenir un médicament liquide et comprenant, en situation d'utilisation, des moyens de sortie associés (13) et des moyens récepteurs (16, 17, 18) adaptés pour recevoir au moins une commande de contrôle depuis la première unité de contrôle et exécuter une action en réponse à celle-ci,
- dans lequel l'unité réservoir et l'unité de contrôle comprennent des moyens de couplage conjugués (14, 24) permettant à une unité de contrôle d'être fixée à l'unité réservoir pour obtenir ainsi une combinaison de celles-ci, la combinaison comprenant des moyens d'expulsion (12) pour expulser un médicament hors du réservoir au travers des moyens de sortie, et un adhésif pour fixer la combinaison sur la peau de l'utilisateur,
- au moins une autre unité de contrôle (30), chaque autre unité de contrôle comprenant un moyen de contrôle produisant une ou plusieurs commandes de contrôle, et des moyens de couplage conjugués (34) permettant à chaque autre unité de contrôle d'être fixée à l'unité réservoir,
- dans lequel les commandes de contrôle comprennent au moins une commande de contrôle contrôlant la vitesse de délivrance ou le profil de délivrance des moyens d'expulsion, dans lequel chaque unité de contrôle est adaptée pour produire une commande de contrôle différente, donnant ainsi pour chaque combinaison d'unité réservoir et d'unité de contrôle des possibilités différentes en termes de vitesse de délivrance ou de profil de délivrance des moyens d'expulsion, **caractérisé en ce que**
- les moyens de contrôle présentent une configuration mécanique produisant les commandes de contrôle pour contrôler la vitesse de délivrance ou le profil de délivrance pour les moyens d'expulsion, et
- l'unité réservoir comprend des moyens de contact adaptés pour coopérer avec les moyens de contrôle mécaniques pour en détecter la configuration mécanique.

2. Un système tel que défini dans la revendication 1, dans lequel l'unité réservoir comprend :
- les moyens d'expulsion (12),
- des moyens processeurs locaux (15) produisant un certain nombre de commandes locales,
- des moyens pour exécuter au moins une action en réponse à une commande locale,
dans lequel les au moins première et seconde unités de contrôle comprennent chacune des moyens de contrôle (35, 45) produisant une ou plusieurs commandes de contrôle pour contrôler le fonctionnement des moyens processeurs locaux, dans lequel chaque unité de contrôle est adaptée pour contrôler une commande locale différente ou une combinaison de commandes locales.

3. Un système tel que défini dans la revendication 2, dans lequel l'unité réservoir comprend en outre au moins un élément contrôlable électroniquement (12) associé aux moyens processeurs locaux, les moyens processeurs locaux étant contrôlables par au moins une unité de contrôle.

4. Un système tel que défini dans les revendications 2 ou 3, dans lequel l'unité réservoir comprend :
- des moyens récepteurs locaux (16) coopérant avec les moyens processeurs locaux (15) pour recevoir des commandes de contrôle depuis au moins une unité de contrôle,
- au moins l'une des unités de contrôle comprenant :
- des moyens processeurs de contrôle (35), et
- des moyens d'émission de contrôle (36) coopérant avec les moyens processeurs de contrôle pour émettre des commandes de contrôle vers les moyens récepteurs locaux pour contrôler une commande locale ou une combinaison de commandes locales.

5. Un système tel que défini dans l'une des revendications 2 à 4, dans lequel l'unité réservoir comprend :
- des moyens d'émission locaux (17) coopérant avec les moyens processeurs locaux (15) pour émettre des informations de données vers les moyens récepteurs de contrôle (35) dans au moins une unité de contrôle,
- au moins l'une des unités de contrôle comprenant :
- des moyens récepteurs de contrôle (47) coopérant avec les moyens processeurs de contrôle (45) pour recevoir des informations de données provenant du processeur local.

6. Un système tel que défini dans l'une des revendications 4 ou 5, dans lequel au moins l'un des moyens d'émission (16) et les moyens de réception correspondants (36) sont adaptés pour une transmission sans fil de commande ou d'information de données.

7. Un système tel que défini dans l'une des revendications 2 à 6, dans lequel les moyens de couplage conjugués incluent des moyens de communication (18, 28) permettant à des informations d'être transférées.

8. Un système tel que défini dans l'une des revendications 1 à 7, comprenant au moins une autre unité réservoir.

9. Un système tel que défini dans l'une des revendications précédentes, dans lequel au moins l'une des unités comprend en outre des moyens d'affichage (268) associés aux moyens processeurs (145) et contrôlés par ceux-ci.

10. Un système tel que défini dans l'une des revendications précédentes, dans lequel au moins une unité réservoir est en forme d'une unité pré-remplie jetable.

11. Un système tel que défini dans l'une des revendications précédentes, dans lequel au moins une unité réservoir comprend un capteur, de préférence associé à des moyens processeurs locaux.

12. Un système tel que défini dans l'une des revendications précédentes, dans lequel au moins une unité réservoir comprend une surface (203) configurée pour être disposée contre une surface de peau d'un utilisateur.

13. Un système tel que défini dans l'une des revendications précédentes, comprenant en outre un capteur pour mesurer de façon continue une pression associée aux moyens de sortie.

14. Un système tel que défini dans l'une des revendications précédentes, comprenant en outre un capteur pour détecter un état dans le corps de l'utilisateur, et des moyens pour contrôler la délivrance du médicament en réponse à cela.

15. Un système tel que défini dans l'une des revendications précédentes, dans lequel au moins une unité de contrôle comprend des moyens mémoire.
